(19) Europäisches Patentamt / European Patent Office / Office européen des brevets

(11) **EP 2 553 457 B1**

(12) **EUROPEAN PATENT SPECIFICATION**

(45) Date of publication and mention
of the grant of the patent:
**11.07.2018 Bulletin 2018/28**

(51) Int Cl.:
***G01N 33/543*** (2006.01)

(21) Application number: **11763142.4**

(22) Date of filing: **29.03.2011**

(86) International application number:
**PCT/SE2011/050343**

(87) International publication number:
**WO 2011/123029 (06.10.2011 Gazette 2011/40)**

(54) **METHOD FOR DETERMINATION OF BINDING STOICHIOMETRY**

VERFAHREN ZUR BESTIMMUNG EINER BINDUNGSSTÖCHIOMETRIE

PROCÉDÉ DE DÉTERMINATION DE LA STOECHIOMÉTRIE DE LIAISON

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.03.2010 SE 1050302**

(43) Date of publication of application:
**06.02.2013 Bulletin 2013/06**

(73) Proprietor: **GE Healthcare Bio-Sciences AB
75184 Uppsala (SE)**

(72) Inventor: **KARLSSON, Robert
S-751 84 Uppsala (SE)**

(74) Representative: **Brann AB et al
P.O. Box 3690
Drottninggatan 27
103 59 Stockholm (SE)**

(56) References cited:
- **DAY ET AL.: 'Selectivity of BAFF/BLyS and APRIL for binding to the TNF family receptors BAFFR/BR3 and BCMA' BIOCHEMISTRY vol. 44, no. 6, 2005, pages 1919 - 1931, XP055094861**
- **LIN ET AL.: 'Determination of binding constant and stoichiometry for antibody-antigen interaction with surface plasmon resonance' CURRENT PROTEOMICS vol. 3, no. 4, 2006, pages 271 - 282, XP055094864**
- **WANG ET AL.: 'A novel spectroscopic titration method for determining the dissociation constant and stoichiometry of protein-ligand complex' ANALYTICAL BIOCHEMISTRY vol. 206, no. 2, 1992, pages 376 - 381, XP024823943**
- **CHU ET AL.: 'Using affinity capillary electrophoresis to determine binding stoichiometries of protein-ligand interactions' BIOCHEMISTRY vol. 33, no. 35, 1994, pages 10616 - 10621, XP055094867**
- **Pascale M. Richalet-Sécordel ET AL: "Concentration Measurement of Unpurified Proteins Using Biosensor Technology under Conditions of Partial Mass Transport Limitation", Analytical Biochemistry, vol. 249, no. 2, 1 July 1997 (1997-07-01), pages 165-173, XP055104164, ISSN: 0003-2697, DOI: 10.1006/abio.1997.2183**

**Description**

**Field of the invention**

The present invention relates to a method for the determination of stoichiometry of binding between two binding partners, such as, for example, a receptor and a ligand.

**Background of the invention**

The number of binding sites involved when two molecules interact, i.e. the binding stoichiometry, is many times of fundamental interest, since it is related to molecular function. The binding stoichiometry can be measured directly, for instance by determining the molecular weight of the formed complex, or it can be measured by indirect methods provided that the concentrations of both interacting molecules, or binding partners, are known.

Prior art indirect methods for determining stoichiometry of binding typically use spectrophotometric methods, such as UV or NIR absorption spectrometry, or fluorescence-based detection for determining the total concentration of a molecule.

US 6,025,142 A discloses determination of the stoichiometry and affinity of the binding of the fluorophore 8-anilino-1-naphthalene sulfonate (ANS) to urokinase-type plasminogen activator (u-PA) by titrating fixed concentrations of u-PAR with ANS up to a concentration of 100 $\mu$m. The theoretical fluorescence of a molar solution if all were bound to u-PAR was calculated by titration of a protein concentration sufficiently high to ensure that all added ANS is bound in the initial part of the binding curve. The generated data were analyzed by the method of Scatchard.

US 2005-0037377 A discloses a method for determining the binding affinity and/or stoichiometry of a binding complex between a binding factor and a probe using fluorescence techniques. The method comprises: (a) labeling the probe with a fluorophore; (b) incubating the labeled probe with a factor or a group of factors which may bind the labeled probe to form a binding complex; (c) separating the binding complex and the free probe into different fractions; (d) subjecting each fraction from step (c) to fluorescence polarization measurement under conditions wherein the binding complex produces a fluorescence pattern different from that of the free probe, thereby allowing detection of the binding complex; and (e) determining binding affinity and/or stoichiometry between the probe and the binding factor. Typically, the binding complex formation is monitored by fluorescence polarization detection.

Zhi-Xin Wang, et al., Anal. Biochem. 206 (1992): 376-381 discloses a titration protocol for determining the dissociation constant and binding stoichiometry of a protein-ligand complex, detectable by spectroscopic methods. In this procedure, a fixed concentration of protein (or ligand) is titrated by increasing volumes of a stock ligand (or protein) solution, and the changes in the spectroscopic signal are recorded after each addition of the titrant. The signal for interaction between protein and ligand first increases, reaches a maximum value, and then starts decreasing due to dilution effect. The volume of the titrant required to achieve the maximum signal changes is utilized to calculate the dissociation constant and the binding stoichiometry of the protein-ligand complex according to the theoretical relationships developed herein. Specifically, the interaction of avidin with a chromophoric biotin analogue, 2-(4'-hydroxyazobenzene)benzoic acid, was studied by following the absorption signal of their interaction at 500 nm.

These methods do, however, not distinguish between active and inactive molecules. Since only active molecules contribute to binding in the interaction studied, total molecule concentration will therefore only provide an estimate of the total concentration of a molecule which may differ substantially from the concentration of "active" molecules. As is readily seen, this can be a substantial dilemma when determining binding stoichiometry by indirect methods.

Other prior art methods within this field are disclosed by Day et al., Biochemistry 44 (2005): 1919-1931; by Lin et al., Current Proteomics 3 (2006): 271-282; and by Chu et al., Biochemistry 33 (1994): 10616-10621.

It is an object of the present invention to provide a method which overcomes the deficiences of the prior art methods and provides for accurate determination of binding stoichiometry by indirect methods even in case of molecule solutions which may contain substantial amounts of inactive interactant molecules.

**Summary of the invention**

According to the present invention, binding stoichiometry is determined based on determination of active molecule concentrations rather than total molecule concentrations.

The method of the present invention for determining binding stoichiometry is defined in independent claim 1.

The method comprises the steps of:

a) preparing a solution of the first molecular species and the second molecular species having predetermined initial active concentrations of the respective molecular species;
b) determining the free active concentration of the first molecular species;

c) determining the free active concentration of the second molecular species;

d) determining the ratio of the difference between the initial active concentration and the free active concentration of the second molecular species to the difference between the initial active concentration and the free active concentration of the first molecular species, or *vice versa;* and

e) determining from said ratio the binding stoichiometry for the interaction.

Determination of active concentration is performed using an interaction analysis sensor, which typically comprises a sensing surface supporting a specific binding partner to the molecular species whose active concentration is to be determined. After contacting the sensing surface with the molecular species, the association/dissociation process at the surface is monitored.

The determination of at least initial active concentration comprises contacting the solution with a sensor surface at varying flow rates under conditions of at least partial mass transport limitation, whereby the use of a calibration standard will not be required.

Further preferred embodiments of the invention are set forth in the dependent claims.

A more complete understanding of the present invention, as well as further features and advantages thereof, will be obtained by reference to the following detailed description and the accompanying drawings.

## Brief description of the drawings

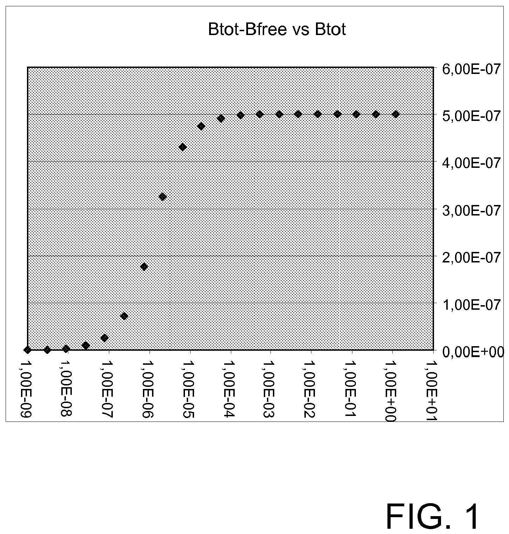

Figure 1 is a diagram showing a plot of (Btot - Bfree) versus Btot for a simulated procedure according to an embodiment of the method of the present invention.

## Detailed description of the invention

Unless defined otherwise, all technical and scientific terms used herein have the same meaning as commonly understood by a person skilled in the art related to this invention. Also, the singular forms "a", "an", and "the" are meant to include plural reference unless it is stated otherwise.

As mentioned above, the present invention relates to the determination of the stoichiometry of binding between two interacting molecules, for example a receptor and a ligand, such that a complex between the molecules is formed. In brief, the method is based on determining initial active concentrations of the interacting molecules and active concentrations of free (non-complexed) molecules of one or both molecules after complex formation has been initiated, and based on the resulting data determining the binding stoichiometry for the interaction.

Further examples of interacting molecule pairs include antibody/antigen.

In one embodiment, the determination of the stoichiometry of the binding between two molecules A and B which may interact to form a complex AB comprises the following steps:

1) incubation of molecules A and B in solution with a fixed initial active concentration of one binding partner (Atot) that is titrated with varying initial active concentrations of the other binding partner (Btot);

2) determination of active concentration of free binding partner B (Bfree) in each mixture;

3) identification of the saturation value(s) for (Btot-Bfree) from plots of (Btot-Bfree) vs Btot, (Btot-Bfree) by definition being the concentration of B molecules in complex with A;

4) determination of the number of binding sites on A for B from the expression $(Btot-Bfree)_{at\ saturation}$/Atot.

Of course, in the procedure outlined above, molecules A and B are interchangeable, i.e., instead, molecule B may be in a fixed active concentration and titrated with varying active concentrations of molecule A.

In another embodiment, which assumes that Afree and Bfree can both be accurately determined for any interaction mixture, the stoichiometry can be determined by studying the ratio of molecules in the complex formed when incubating fixed concentrations of molecules A and B in solution, i.e. by calculating the ratio (Btot-Bfree)/(Atot-Afree). This will provide a "snapshot" of complex stoichiometry for these conditions which, however, may differ from the step determination since sites of different affinity on molecule A need not be populated at the same time. Optionally, measurements may be performed on two or more different mixtures of molecules A and B to obtain a more accurate stoichiometry value.

The determination of active concentration of molecules A and B is preferably performed using an interaction analysis sensor, typically a biosensor. Such biosensor-based determination of active concentration is described in, for example Karlsson, R., et al. (1993) J. Immunol. Methods 166(1):75-84; Richalet-Secordel, P. M., et al. (1997) Anal Biochem. 249(2):165-73; and Sigmundsson K., et al. (2002) Biochemistry 41(26):8263-76. The interaction analysis sensor typically comprises a sensing surface(s) having immobilized thereon a specific binding partner for the molecule whose active concentration is to be determined.

In a development of the determination of active concentration using sensor technology, analyte concentrations can be determined without reference to a calibration standard. This method, which is usually referred to as Calibration-Free Concentration Analysis (CFCA), relies upon measurement of analyte binding to a target immobilized on a sensor surface at varying flow rates under conditions where the observed rate of binding is partially or completely limited by transport of analyte molecules to the sensor surface, i.e. partially or completely controlled by diffusion. CFCA will be described in more detail further below. First, however, the concept of biosensors will be briefly described.

A biosensor is typically based on label-free techniques, detecting a change in a property of a sensor surface, such as mass, refractive index or thickness of the immobilized layer. Typical biosensors for the purposes of the present invention are based on mass detection at the sensor surface and include especially optical methods and piezoelectric or acoustic wave methods. Representative sensors based on optical detection methods include those that detect mass surface concentration, such as sensors based on reflection-optical methods, including e.g. evanescent wave-based sensors including surface plasmon resonance (SPR) sensors, frustrated total reflection (FTR) sensors, and waveguide sensors, including e.g. reflective interference spectroscopy (RIfS) sensors. Piezoelectric and acoustic wave sensors include surface acoustic wave (SAW) and quartz crystal microbalance (QCM) sensors.

Biosensor systems based on SPR and other detection techniques are commercially available today. Exemplary such SPR-biosensors include the flow-through-cell-based Biacore® systems (GE Healthcare Bio-Sciences AB, Uppsala, Sweden) and ProteOn™ XPR system (Bio-Rad Laboratories, Hercules, CA, USA) which use surface plasmon resonance for detecting interactions between molecules in a sample and molecular structures immobilized on a sensing surface or surfaces. As sample is passed over the sensing surface, the progress of binding directly reflects the rate at which the interaction occurs. Injection of sample is usually followed by a buffer flow during which the detector response reflects the rate of dissociation of the complex on the surface. A typical output from the system is a graph or curve describing the progress of the molecular interaction with time, including an association phase part and a dissociation phase part. This binding curve, which is usually displayed on a computer screen, is often referred to as a "sensorgram".

With the Biacore® systems it is thus possible to determine in real time without the use of labeling, and often without purification of the substances involved, not only the presence and concentration of a particular molecule, or analyte, in a sample, but also additional interaction parameters, including kinetic rate constants for association (binding) and dissociation in the molecular interaction as well as the affinity for the surface interaction.

In the following, the present invention will to a large extent be described, for illustration only and no limitation, with regard to SPR-sensors of the Biacore® system type.

The Biacore® systems, as well as analogous sensor systems, measure the active analyte concentration as distinct from the total concentration of the analyte. As to the term "active", it is the choice of ligand on the sensor surface that defines the kind of activity being measured. While e.g. standard protein concentration analysis using a calibration curve may be used, the Biacore® systems (and analogous sensor systems) permit assessment of protein (and e.g. other macromolecule) concentration by a calibration-free method, which is often referred to as calibration-free concentration analysis (CFCA).

The method relies on changes in binding rates of analyte to a target (ligand) immobilized on a surface with varying flow rates under conditions of partial or total mass transport and does, as mentioned, not require standards of known concentrations, given that the diffusion coefficient is known or is estimated from the molecular mass of the molecule of interest. For a more detailed description such calibration-free measurement it may be referred to, for example, the above-mentioned Sigmundsson, K., et al. (2002) Biochemistry 41(26): 8263-8276.

In Biacore® instruments, or analogous instruments, samples are injected in a micro-flow system and transported in a laminar flow to the sensor surface. Molecules reach the sensor surface from bulk solution by a diffusion-controllled transport process. In addition to the concentration of analyte molecules, factors influencing the transport include the diffusion coefficient, flow cell dimensions and flow rate. The balance between the transport rate and the binding rate determines whether the observed binding will be transport limited or reaction limited.

For successful CFCA, the observed binding rate must be at least partially limited by transport. The concentration is obtained by running the binding experiments at at least two different flow rates and fitting the data to a model describing the process, e.g. a two-compartment model (Myszka, D. G., et al. (1998) Biophys. J. 75, 583-594, and Schank-Retzlaff, M. L. and Sligar, S. G. (2000) Anal. Chem. 72, 4212-4220). For a more comprehensive description of curve fitting with regard to the Biacore® systems, it may be referred to the BIAevaluation™ Software Handbook (GE Healthcare Bio-Sciences AB, Uppsala, Sweden).

The binding of analyte to surface-attached ligand in a controlled flow system is represented by the sum of two processes, transport of analyte to the surface and molecular interaction with the immobilized ligand. The molecular interaction is described by the rate constants $k_a$ and $k_d$, while transport of analyte to and from the surface is described by the mass transport constants $k_m$ and $k_{-m}$ (also referred to as $k_t$ and $k_{-t}$). The transport phenomenon is symmetrical since this is essentially a diffusion-limited process, so $k_m = k_{-m}$.

Thus, for determining active concentration of, for example, a protein using a Biacore® system (or analogous), a protein solution is injected at least twice (different flow rates) over the surface with immobilized interaction partner.

The binding phases of the sensorgrams obtained from such an experiment are fitted to a bi-molecular interaction model with mass transfer term, in which the active concentration is a fitted parameter. The fitting is preferably global, i.e. the interaction model is fitted simultaneously to multiple binding curves (sensorgrams). In this model, the value of the mass transport coefficient is introduced as a constant, which, as described above, may be calculated from the dimensions of the flow cell, the diffusion coefficient of the protein and the flow rate used.

In a simplified form, the response increase dR/dt at the sensor surface given by bound protein is proportional to the mass transport constant $k_t$ and the active concentration, i.e.

$$dR/dt = k_t*(\text{active concentration}) \qquad (2)$$

$k_t$ can be re-written as constant$*Mw*D^{2/3}$, where Mw is the molecular weight of the protein and D is its diffusion coefficient, which gives

$$dR/dt = \text{constant}*Mw*D^{2/3}*(\text{active concentration}) \qquad (3)$$

The diffusion coefficient D is a function of the size and shape of the molecule and the frictional resistance offered by the viscosity of the solvent in question. For spherical molecules, the diffusion coefficient is inversely proportional to the radius and thus proportional to the cube root of the molecular weight. For very large solute molecules, such as proteins, however, the diffusion coefficient is relatively insensitive to the molecular weight.

Now turning to the present invention again, to measure active concentrations of the molecules A and B with a Biacore™ type sensor instrument, specific binding partners to the respective molecules are provided for immobilization on a sensing surface of the sensor instrument.

To carry out the above first-mentioned method variant involving titration, respective stock solutions containing molecules A and B are prepared, and the active concentrations of molecules A and B are determined using CFCA and sensing surfaces with immobilized binding partner to molecules A and B, respectively. A number of solution mixtures are then prepared from the stock solutions which contain a fixed concentration of molecule A and varying concentrations of the molecule B. The initial concentrations of molecules A and B (Atot and Btot, respectively), i.e. the concentrations before any interaction has taken place, may be calculated from the volumes of stock solutions used. After incubation, the active concentrations of molecule B in the different mixtures are determined using a sensing surface(s) with immobilized binding partner to molecule B and either (i) CFCA, or (ii) a standard or calibration curve (prepared using the active concentration determined by CFCA for the stock solution of B). Based on the results of the concentration measurements, the binding stoichiometry for the molecular interaction may then be determined as described further above.

When titrating a fixed active concentration of one binding partner with varying active concentrations of the other binding partner as described above, the different solution mixtures are prepared before being injected into the biosensor instrument, or, optionally, solutions of the respective interactants in known active concentrations may be injected into the biosensor instrument to be mixed in predetermined proportions within the instrument, as described in, for example, WO 2008/033073.

The above-mentioned other method variant, including determination of free active concentrations of molecules A and B after incubation, may be performed in an analogous manner.

In the following Example, a simulation of a procedure for the determination of binding stoichiometry according to the method of the present invention will be described.

**Example**

A simulation of the stoichiometry for the binding interaction between two molecules A and B (forming a complex AB) is presented in Table 1 below. The input data were as follows:

|  |  |
|---|---|
| Total concentration of A: | 5,00E-07 |
| Total concentration of B: | 1,00E-09 |
| Affinity: | 1,00E-06 |

Table 1

| A total (M) | B total (M) | KD | Complex formed | Free A | Atot-Afree | Free B | Btot-Bfree | (Atot-Afree)/(Btot-Bfree) |
|---|---|---|---|---|---|---|---|---|
| 5,00E-07 | 1,00E-09 | 1.00E-06 | 3,33E-10 | 5,00E-07 | 3,33E-10 | 6,67E-10 | 3,33E-10 | 1,0 |
| 5,00E-07 | 3,00E-09 | 1.00E-06 | 9,99E-10 | 4,99E-07 | 9,99E-10 | 2,00E-09 | 9,99E-10 | 1,0 |
| 5,00E-07 | 9,00E-09 | 1.00E-06 | 2,99E-09 | 4,97E-07 | 2,99E-09 | 6,01E-09 | 2,99E-09 | 1,0 |
| 5,00E-07 | 2,70E-08 | 1.00E-06 | 8,89E-09 | 4,91E-07 | 8,89E-09 | 1,81E-08 | 8,89E-09 | 1,0 |
| 5,00E-07 | 8,10E-08 | 1.00E-06 | 2,6E-08 | 4,74E-07 | 2,60E-08 | 5,50E-08 | 2,60E-08 | 1,0 |
| 5,00E-07 | 2,43E-07 | 1.00E-06 | 7,27E-08 | 4,27E-07 | 7,27E-08 | 1,70E-07 | 7,27E-08 | 1,0 |
| 5,00E-07 | 7,29E-07 | 1.00E-06 | 1,78E-07 | 3,22E-07 | 1,78E-07 | 5,51E-07 | 1,78E-07 | 1,0 |
| 5,00E-07 | 2,19E-06 | 1.00E-06 | 3,25E-07 | 1,75E-07 | 3,25E-07 | 1,86E-06 | 3,25E-07 | 1,0 |
| 5,00E-07 | 6,56E-06 | 1.00E-06 | 4,3E-07 | 7,01E-08 | 4,30E-07 | 6,13E-06 | 4,30E-07 | 1,0 |
| 5,00E-07 | 1,97E-05 | 1.00E-06 | 4,75E-07 | 2,47E-08 | 4,75E-07 | 1,92E-05 | 4,75E-07 | 1,0 |
| 5,00E-07 | 5,90E-05 | 1.00E-06 | 4,92E-07 | 8,40E-09 | 4,92E-07 | 5,86E-05 | 4,92E-07 | 1,0 |
| 5,00E-07 | 1,77E-04 | 1.00E-06 | 4,97E-07 | 2,81E-09 | 4,97E-07 | 1,77E-04 | 4,97E-07 | 1,0 |
| 5,00E-07 | 5,31E-04 | 1.00E-06 | 4,99E-07 | 9,40E-10 | 4,99E-07 | 5,31E-04 | 4,99E-07 | 1,0 |
| 5,00E-07 | 1,59E-03 | 1.00E-06 | 5E-07 | 3,14E-10 | 5,00E-07 | 1,59E-03 | 5,00E-07 | 1,0 |
| 5,00E-07 | 4,78E-03 | 1.00E-06 | 5E-07 | 1,05E-10 | 5,00E-07 | 4,78E-03 | 5,00E-07 | 1,0 |
| 5,00E-07 | 1,43E-02 | 1.00E-06 | 5E-07 | 3,48E-11 | 5,00E-07 | 1,43E-02 | 5,00E-07 | 1,0 |
| 5,00E-07 | 4,30E-02 | 1.00E-06 | 5E-07 | 1,16E-11 | 5,00E-07 | 4,30E-02 | 5,00E-07 | 1,0 |
| 5,00E-07 | 1,29E-01 | 1.00E-06 | 5E-07 | 3,87E-12 | 5,00E-07 | 1,29E-01 | 5,00E-07 | 1,0 |
| 5,00E-07 | 3,87E-01 | 1.00E-06 | 5E-07 | 1,29E-12 | 5,00E-07 | 3,87E-01 | 5,00E-07 | 1,0 |
| 5,00E-07 | 1,16E+00 | 1.00E-06 | 5E-07 | 4,30E-13 | 5,00E-07 | 1,16E+00 | 5,00E-07 | 1,0 |

Using values from the simulation data above, Btot-Bfree was plotted against Btot, the resulting graph being shown in Figure 1. As is readily seen, in an unknown case (i.e. KD and binding mechanism are unknown), such a plot will reveal binding stoichiometry (in the illustrated case 1:1).

A determination of stoichiometry according to the invention may, for example, be performed using a Biacore™ system, e.g. a Biacore® T100 (GE Healthcare Bio-Sciences AB, Uppsala, Sweden), wherein a micro-fluidic system passes samples and running buffer through four individually detected flow cells (one by one or in series).

As sensor chip may, for example, be used Series S Sensor Chip CM5 (GE Healthcare Bio-Sciences AB) which has a gold-coated surface with a covalently carboxymethyl-modified dextran polymer hydrogel. The output from the instrument is a "sensorgram" which is a plot of detector response (measured in "resonance units", RU) as a function of time. An increase of 1000 RU corresponds to an increase of mass on the sensor surface of approximately 1 ng/mm$^2$.

For calculations, the dedicated BIAevaluation Software and Biacore T100 Software 2.0 (GE Healthcare Bio-Sciences AB, Uppsala, Sweden) may be used, which includes a module for calibration-free concentration analysis (CAFC).

A procedure for determining the number of binding sites on a molecule A for molecule B using a Biacore® T100 may be performed as follows:

**A Determination of active concentrations**

1) Insert sensor chip CM5 and prime the system with buffer.
2) Immobilize molecule A in flow cell 2 and molecule B in flow cell 4.
Aim to immobilize between 25 and 100 RU/kDa. (That is, if molecule A has a molecular weight of 50 kDa, immobilize between 1250 and 5000 RU).
3) Inject molecule B over immobilized ligand A and use a dilution that gives an initial binding rate of at least 0,3 RU/s at a flow rate of 5 $\mu$l/min. Use an injection time of 60 s.
4) Inject the same dilution of molecule B at a flow rate of 100 $\mu$l/min also for 60 s.
5) In the same manner as described in steps 3 and 4, inject molecule A over immobilized ligand B at 5 and 100 $\mu$l/min.
6) Open T100 evaluation software and determine the concentration of molecules A and B with the analysis tool Concentration analysis/Calibration free.

**B) Determination of stoichiometry**

With knowledge of the active concentration of molecules A and B, the stoichiometry of binding can now be determined.

1) Use a fix concentration "2a" of molecule A and pipette 100 $\mu$l of that solution into at least 10 wells of a 96 well plate.
2) Prepare a 200 $\mu$l solution of molecule B at concentration 100*2a (or higher) and prepare 10 (or more) successive three-fold dilutions of B.
These solutions are to be used in a standard curve for determination of the free concentration of B and for incubation with molecule A.
3) Transfer 100 $\mu$l of each B solution to a well where molecule A is already present at concentration "2a", giving an initial concentration "a" of molecule A.
4) Inject standard concentrations of B over immobilized ligand A.
5) Inject solutions of A incubated with varying concentrations of B over immobilized ligand A.
In steps 4 and 5, a typical injection time could be 3 minutes and a typical flow rate 10 $\mu$l/min. Use a report point set 10 seconds after injection stop as response value.
6) Open T100 evaluation software and prepare a standard curve for B by plotting the response value obtained from the injection versus the known concentration of B.
7) Determine the free concentration of B in each mixture.
8) Plot (Btot-Bfree) vs Btot and use data points at saturation (see Fig 1) to calculate the stoichiometry from the expression stochiometry = **(Btot-Bfree)**$_{\text{at saturation}}$**/Atot.**

The present invention is not limited to the above-described preferred embodiments. Therefore, the above embodiments should not be taken as limiting the scope of the invention, which is defined by the appending claims.

**Claims**

**1.** A method of determining binding stoichiometry for the interaction between a first molecular species and a second molecular species forming a complex between them, comprising the steps of:

b1) preparing a solution of the first molecular species and the second molecular species having predetermined initial active concentrations of the respective molecular species,
b2) determining the free active concentration of the first molecular species,
b3) determining the free active concentration of the second molecular species,
b4) determining the ratio of the difference between the initial active concentration and the free active concentration of the second molecular species to the difference between the initial active concentration and the free active concentration of the first molecular species, and

EP 2 553 457 B1

b5) determining from said ratio the binding stoichiometry for the interaction,

and

wherein active concentrations of said molecular species are determined using an interaction analysis sensor comprising a sensing surface supporting a specific binding partner to the molecular species whose active concentration is to be determined, and wherein the determination of the active concentrations comprises contacting a solution containing the molecule to be determined with a sensor surface at varying flow rates under conditions of at least partial mass transport limitation and is performed without the use of a calibration standard.

2. The method according to claim 1, wherein the interaction analysis sensor is a biosensor.

3. The method according to claim 2, wherein the biosensor is a mass-sensing biosensor, preferably a biosensor based on evanescent wave sensing, especially surface plasmon resonance (SPR).

**Patentansprüche**

1. Verfahren zum Bestimmen der Bindungsstöchiometrie für die Interaktion zwischen einer ersten molekularen Spezies und einer zweiten molekularen Spezies, die einen Komplex zwischen ihnen bilden, umfassend die folgenden Schritte:

b1) Vorbereiten einer Lösung der ersten molekularen Spezies und der zweiten molekularen Spezies mit vorbestimmten anfänglichen aktiven Konzentrationen der jeweiligen molekularen Spezies,
b2) Bestimmen der freien aktiven Konzentration der ersten molekularen Spezies,
b3) Bestimmen der freien aktiven Konzentration der zweiten molekularen Spezies,
b4) Bestimmen des Verhältnisses der Differenz zwischen der anfänglichen aktiven Konzentration und der freien aktiven Konzentration der zweiten molekularen Spezies zur Differenz zwischen der anfänglichen aktiven Konzentration und der freien aktiven Konzentration der ersten molekularen Spezies, und
b5) Bestimmen, aus dem Verhältnis, der Bindungsstöchiometrie für die Interaktion, und

wobei aktive Konzentrationen der molekularen Spezies unter Verwendung eines Interaktions-Analysessensors bestimmt werden, der eine Sensoroberfläche umfasst, die einen spezifischen Bindungspartner an die molekulare Spezies hält, deren aktive Konzentration bestimmt werden soll, und wobei die Bestimmung der aktiven Konzentrationen ein Kontaktieren einer Lösung, die das zu bestimmende Molekül enthält, mit einer Sensorfläche bei variierenden Strömungsraten unter Bedingungen von zumindest einer partiellen Massentransportbeschränkung umfasst und ohne die Verwendung eines Kalibrierungsstandards durchgeführt wird.

2. Verfahren nach Anspruch 1, wobei der Interaktions-Analysesensor ein Biosensor ist.

3. Verfahren nach Anspruch 2, wobei der Biosensor ein massedetektierender Biosensor ist, vorzugsweise ein Biosensor basierend auf Evaneszente-Welle-Detektion, insbesondere Oberflächen-Plasmon-Resonanz (SPR).

**Revendications**

1. Procédé de détermination de la stoechiométrie de liaison pour l'interaction entre une première espèce moléculaire et une seconde espèce moléculaire formant un complexe entre eux, comprenant les étapes de :

b1) préparer une solution de la première espèce moléculaire et de la seconde espèce moléculaire ayant des concentrations actives initiales prédéterminées des espèces moléculaires respectives,
b2) déterminer la concentration active libre des premières espèces moléculaires,
b3) déterminer la concentration active libre de la seconde espèce moléculaire,
b4) déterminer le ratio de la différence entre la concentration active initiale et la concentration active libre de la seconde espèce moléculaire par rapport à la différence entre la concentration active initiale et la concentration active libre de la première espèce moléculaire, et
b5) déterminer à partir dudit ratio la stoechiométrie de liaison pour l'interaction,
et

dans lequel les concentrations actives desdites espèces moléculaires sont déterminées en utilisant un capteur d'analyse d'interaction comprenant une surface de détection servant de support à un partenaire de liaison spécifique aux espèces moléculaires dont la concentration active doit être déterminée, et dans lequel la détermination des concentrations actives comprend la mise en contact d'une solution contenant la molécule à déterminer avec une surface de capteur à des débits variables dans des conditions de limitation de transport de masse au moins partielle et est réalisée sans l'utilisation d'un standard d'étalonnage.

2. Procédé selon la revendication 1, dans lequel le capteur d'analyse d'interaction est un biocapteur.

3. Procédé selon la revendication 2, dans lequel le biocapteur est un biocapteur à détection de masse, de préférence un biocapteur basé sur une détection d'onde évanescente, en particulier une résonance plasmonique de surface (SPR).

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6025142 A
- US 20050037377 A
- WO 2008033073 A

### Non-patent literature cited in the description

- **ZHI-XIN WANG et al.** *Anal. Biochem.,* 1992, vol. 206, 376-381
- **DAY et al.** *Biochemistry,* 2005, vol. 44, 1919-1931
- **LIN et al.** *Current Proteomics,* 2006, vol. 3, 271-282
- **CHU et al.** *Biochemistry,* 1994, vol. 33, 10616-10621
- **KARLSSON, R. et al.** *J. Immunol. Methods,* 1993, vol. 166 (1), 75-84
- **RICHALET-SECORDEL, P. M. et al.** *Anal Biochem.,* 1997, vol. 249 (2), 165-73
- **SIGMUNDSSON K. et al.** *Biochemistry,* 2002, vol. 41 (26), 8263-76
- **SIGMUNDSSON, K. et al.** *Biochemistry,* 2002, vol. 41 (26), 8263-8276
- **MYSZKA, D. G. et al.** *Biophys. J.,* 1998, vol. 75, 583-594
- **SCHANK-RETZLAFF, M. L. ; SLIGAR, S. G.** *Anal. Chem.,* 2000, vol. 72, 4212-4220